(19) Europäisches Patentamt
European Patent Office
Office européen des brevets

(11) **EP 4 417 121 A1**

(12) **EUROPEAN PATENT APPLICATION**

(43) Date of publication:
**21.08.2024 Bulletin 2024/34**

(21) Application number: **23157442.7**

(22) Date of filing: **20.02.2023**

(51) International Patent Classification (IPC):
***A61B 5/08*** (2006.01)          ***A61B 5/021*** (2006.01)
***A61B 5/00*** (2006.01)

(52) Cooperative Patent Classification (CPC):
**A61B 5/0816; A61B 5/02108; A61B 5/7235**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **PFEIFFER, Ulrich**
  **Eindhoven (NL)**
• **STOLZE, Benjamin**
  **Eindhoven (NL)**
• **REGH, Stephan Guido Maria**
  **5656AG Eindhoven (NL)**

(74) Representative: **Philips Intellectual Property &
Standards
High Tech Campus 52
5656 AG Eindhoven (NL)**

(54) **APPARATUS FOR DETERMINING A RESPIRATION RATE OF A SUBJECT**

(57)    The invention relates to an apparatus for determining a respiration rate of a subject. A pressure signal providing unit provides a measured pressure signal of a subject over a time, wherein the measured pressure signal is indicative of blood pulsations and comprises a plurality of pressure pulses, and a processor determines, for a respective pressure pulse of the plurality of pressure pulses 9, a respective dicrotic notch position and determines the respiration rate based on the dicrotic notch positions determined for the plurality of pressure pulses 9. This allows for a very accurate determination of the respiration rate.

FIG. 4

**Description**

FIELD OF THE INVENTION

The invention relates to an apparatus, a method and a computer program for determining a respiration rate of a subject.

BACKGROUND OF THE INVENTION

WO 2011/098763 discloses an apparatus for determining a respiration rate of a subject. The apparatus comprises an input for receiving an arterial pressure waveform and means for defining a start point within the waveform at the beginning of a systolic arterial pressure increase at a ventricular contraction phase of a heartbeat. The apparatus further comprises means arranged to determine an end point of the heartbeat by identifying a part or point of the waveform that has substantially the same arterial pressure value as the starting point, and, if the arterial pressure value at the starting point is not regained, extrapolating the waveform to a point where it has substantially the same arterial pressure value as the starting point. The apparatus also comprises means for calculating a stroke volume from the waveform or the extrapolated waveform between the start point and the end point and for determining a respiration rate based on cyclic changes in the stroke volume.

SUMMARY OF THE INVENTION

It is an object of the present invention to provide an apparatus, a method and a computer program which allow for an improved determination of a respiration rate of a subject.
In a first aspect of the invention an apparatus for determining a respiration rate of a subject is presented, wherein the apparatus comprises

- a pressure signal providing unit configured to provide a measured pressure signal of a subject over a time, wherein the measured pressure signal is indicative of blood pulsations and comprises a plurality of pressure pulses,
- a processor configured to determine, for a respective pressure pulse of the plurality of pressure pulses, a respective dicrotic notch position and to determine the respiration rate based on the dicrotic notch positions determined for the plurality of pressure pulses.

Determining the respiration rate based on the dicrotic notch positions allows to determine the respiration rate very accurately. The processor can determine the respiration rate directly or indirectly based on the dicrotic notch positions. In the latter case the dicrotic notch positions are used for calculating another parameter which is related to the respective pressure pulse and the respiration rate is determined depending on this other parameter. This will be described exemplarily further below.

Moreover, the processor can determine the respective dicrotic notch position directly based on the respective measured pressure pulse or based on a respective processed measured pressure pulse. If the dicrotic notch position is determined based on a processed measured pressure pulse, the processing preferentially is carried out by the processor. However, it also is possible that it is carried out, at least partly, by the pressure signal providing unit.

The measured pressure pulses can be, for instance, non-invasively measured pressure pulses or invasively measured pressure pulses.

The pressure signal providing unit can be a receiving unit configured to receive the measured pressure signal from, for instance, a measurement device and to provide the received pressure signal. However, the pressure signal providing unit can also be a storage in which a previously measured pressure signal has been stored and from which it can be retrieved. The pressure signal providing unit also can be or comprise the measurement device which measures the pressure signal.

In an example, the pressure signal providing unit is configured to provide as the pressure signal a pressure signal that has been measured by using a measurement device comprising a) a shell configured to encase a part of the subject, through which blood flows, b) a pressure applicator configured to apply pressure to the shell and thereby to the encased part of the subject, and c) a pressure sensor configured to measure the pressure signal on the skin of the encased part of the subject, wherein the pressure applicator increases or decreases the applied pressure while the pressure signal is measured. Thus, the pressure signal can be a non-invasively measured pressure signal, wherein nevertheless the respiration rate can be determined with high quality. However, the pressure signal providing unit also can be configured to provide an invasively measured pressure signal, as mentioned above.

In the example with the measurement device comprising the shell, the pressure applicator and the pressure sensor, the pressure sensor can be arranged inside the shell. However, the pressure sensor can also be arranged in another way for measuring the pressure on the skin of the encased part of the subject. For instance, a fluid filled pressure sensor pad can be arranged inside the shell and connected via a fluid path, i.e. via a fluid filled tubing, to a pressure sensor outside of the shell, in order to measure the pressure on the skin of the encased part of the subject.

The processor can be configured to determine, for the respective pressure pulse, a respiration rate determination value based on the determined dicrotic notch position such that for several pressure pulses, which are present at different times, several respiration rate determination values are determined, wherein the several respiration rate determination values determined for the several pressure pulses and hence for several times form a respiration rate determination curve, and to determine the respiration rate based on the respiration rate determination curve.

In particular, the processor can be configured to, for a respective pressure pulse, determine at least one feature that depends on the determined dicrotic notch position and determine the respective respiration rate determination value based on the determined at least one feature, particularly without basing the determination of the respective respiration rate determination value on further features which characterize the respective pressure pulse and which do not depend on the determined dicrotic notch position. However, the processor also can be configured to, for a respective pressure pulse, determine at least one additional feature that characterizes the respective pressure pulse and that does not necessarily depend on the dicrotic notch position and determine the respiration rate also based on the at least one additional feature. In particular, the processor can be configured to determine, for the respective pressure pulse, the respiration rate determination value based on several features, wherein at least one of these features is based on the determined dicrotic notch position and at least one additional feature also characterizes the respective pressure pulse, but is not necessarily based on the dicrotic notch position, such that for several pressure pulses, which are present at different times, several respiration rate determination values are determined, wherein the several respiration rate determination values determined for the several pressure pulses and hence for the different times form the respiration rate determination curve. As mentioned above, the processor can be configured to determine the respiration rate based on the respiration rate determination curve.

The processor can be adapted to process the several respiration rate determination values, which are obtained for the several pressure pulses, for obtaining a continuous respiration rate determination curve. For instance, an interpolation can be applied and optionally also a smoothing.

In an example, the processor is configured to, in order to determine for a respective pressure pulse a respective respiration rate determination value, determine at least one of a) the area under the respective pressure pulse between i) a first position being a start position, at which the respective pressure pulse starts, or being a position between the start position and determined dicrotic notch position, and ii) the determined dicrotic notch position, and b) the difference between the first position and the dicrotic notch position. It has been found that by using at least one of these features, i.e. the previously mentioned area and/or the previously mentioned difference, the determination of the respiration rate can be further improved.

In particular, the processor can be configured such that the respective respiration rate determination value is determined by applying a predefined function to at least one of a) the area under the respective pressure pulse between the first position and the determined dicrotic notch position, and b) the difference between the first position and the dicrotic notch position. For instance, the processor can be configured such that the respective respiration rate determination value is identical to or linearly related, particularly proportional, to the area under the respective pressure pulse between the first position and the determined dicrotic notch position. In other words, in a preferred example the predefined function is the identity function or a linear function, particularly a proportional function.

In an example, the processor is configured to determine the respiration rate by determining a frequency of the respiration rate determination curve, preferentially the main frequency of the respiration rate determination curve, particularly if the respiration rate determination curve has been determined based on at least one of the area and difference features described in the previous paragraph.

As explained above, the respective pressure pulse, of which the dicrotic notch is to be determined, can be directly a measured pressure pulse or a processed measured pressure pulse, wherein the measured pressure pulse can be a pressure pulse of a plurality of pressure pulses of a measured pressure signal. The respective pressure pulse can be processed by, for instance, subtracting a mean measured pressure from the respective pressure pulse. However, the pressure pulse can also be processed in another way. Also the determination of the respiration rate determination values, particularly of the features that characterize the respective pressure pulse like the area under the respective pressure pulse between a start position, at which the respective pressure pulse starts, and the determined dicrotic notch position, can be carried out directly with the measured pressure pulses or with the processed measured pressure pulses.

In an example, the processor is configured to apply a frequency range pre-selecting filter to the respiration rate determination curve which might also be regarded as being a respiration rate determination signal. This filter is preferentially configured such that a predetermined frequency range, in which the respiration rate could be present, is not or substantially not affected. For instance, this filter can be a frequency filter which reduces or eliminates frequency components outside of the predetermined frequency range. The frequency range pre-selecting filter preferentially is a band pass filter which can be a combination of a low pass filter and a high pass filter.

In an example, the processor is configured to determine the dicrotic notch position by determining at least one of a) a notch determination intersection position being a position at which a shifted tangent intersects with the pressure pulse, wherein the shifted tangent is determined by determining a tangent to the pressure pulse at a position of the pressure pulse, at which the first derivative of the pressure pulse has a minimum, and by shifting the tangent in the direction of increasing time by a shifting distance, b) a notch determination maximum position being a position at which a derivative function has a maximum, wherein the derivative function is determined by determining the first derivative of the pressure pulse, by determining the second derivative of the pressure pulse and by combining the determined first and second derivatives, and c) a notch determination function position that is determined by determining a characteristic of the pressure pulse and by applying a position determination function to the characteristic, wherein the position determination function provides a relation between the characteristic and the notch determination function position. A processor configured in this way allows for an improved determination of the dicrotic notch position and hence of the respiration rate based on the dicrotic notch position.

In accordance with the explanation given above, the pressure pulse, to which the determination of at least one of the notch determination intersection position, the notch determination maximum position and the notch determination function position is applied, can directly be a measured pressure pulse or a processed measured pressure pulse. The minimum of the first derivative of the pressure pulse preferentially is the global minimum of the first derivative of the pressure pulse.

In an example, the processor is configured to provide the shifting distance such that it is dependent on a characteristic of the pressure pulse. The characteristic can be any characteristic that is related to the pressure pulse and the shifting distance can depend on one or several characteristics. For instance, the shifting distance can depend on at least one of a) the pulse rate, which could also be named heart rate, b) the position of the minimum of the first derivative of the pressure pulse in between the pressure pulse's maximum systolic pressure and the pressure pulse's pressure at the end-diastolic point and/or in between the time of the pressure pulse's maximum systolic pressure and the time at the end of the pressure pulse, c) the minimum value of the first derivative and d) the width of the pressure pulse at a predefined percentage of the difference between the pressure pulse's maximum systolic pressure and the pressure pulse's pressure at the end-diastolic point. The predefined percentage preferentially is within a range from 50 % to 80 %. In a preferred embodiment the predefined percentage is 66 %. In an example, the width of the pressure pulse at the predefined percentage of the difference between the pressure pulse's maximum systolic pressure and the pressure pulse's pressure at the end-diastolic points divided by the full pulse length, i.e. by the pulse duration, in order to determine the shifting distance. In particular, the resulting ratio, which might be named pulse width ratio, can be the input of a shifting distance function which provides as output the shifting distance. For instance, the shifting distance can be calculated in accordance with following equation:

$$\text{Shifting distance} = (\text{pulse width ratio} - m) * n \quad , \qquad (1)$$

wherein m and n are parameters that are predetermined by calibration.

Hence, the dependence of the shifting distance on one or several characteristics of the respective pulse can be determined in a calibration procedure, wherein the dependence of the shifting distance on the one or several characteristics of the respective pulse is determined such that the determined intersection position corresponds to the position of the dicrotic notch that is known during this calibration procedure. It has been found that a dependence defining, the higher the position of the minimum of the first derivative in between the pressure pulse's maximum systolic pressure and the pressure pulse's pressure at the end-diastolic point, the larger the shifting distance, provides good results. It further has been found that a dependence defining, the larger the pulse rate, the smaller the shifting distance, or that the shifting distance decreases with increasing pulse rate, also provides good results. The shifting distance can also be constant. It has been found that already a constant shifting distance can lead to a high quality determination of the dicrotic notch position, wherein the quality of determining the dicrotic notch position can be further improved, if the shifting distance depends on one or several characteristics of the pressure pulse.

The constant shifting distance can be predetermined by statistical analysis of a training data set which is used during a calibration procedure and which comprises already known dicrotic notch positions, the tangents and hence the shifting distances which are required to shift the tangents such that the resulting intersection positions coincide with the known dicrotic notch positions. In an embodiment, the statistical analysis includes calculating the average of the shifting distances found while carrying out the calibration procedure. In a preferred embodiment, the constant shifting distance is within a range from 10 ms to 50 ms. Preferentially, the constant shifting distance is 20 ms.

In an example, the processor is configured to, if several intersection positions of the shifted tangent with the pressure pulse exist, select the temporally last intersection position as the notch determination intersection position. It has been found that this allows for a further improved accuracy of determining the intersection position such that it corresponds to the position of the dicrotic notch.

Moreover, in an example, the processor is configured to determine the notch determination maximum position by determining whether the highest maximum of the derivative function is larger than the second highest maximum multiplied by a predefined factor, wherein, if this is the case, the notch determination maximum position is the position of the highest maximum and, if this is not the case, the notch determination maximum position is the position of the first maximum of the derivative function, wherein preferentially the processor is configured to consider only maxima of the derivative function being larger than a first predefined derivative threshold and/or being within a provided search range. The first maximum of the derivative function is the temporally first, i.e. the earliest, maximum of the derivative function. This allows to determine the notch determination derivative position such that it even more accurately corresponds to the position of the dicrotic notch of the pressure pulse.

In an example, the processor is configured to determine that the pressure pulse has an artifact, if the derivative function comprises at least two maxima, particularly in the search range, being larger than a second predefined derivative threshold, wherein the second predefined derivative threshold is larger than the first predefined derivative threshold. If the derivative function of the respective pulse comprises at least two maxima being larger than the first predefined derivative threshold, the derivative function has at least two very large maxima, wherein this should not be the case if a regular pressure pulse were present. In other words, if at least two very large maxima are present, an artifact is very likely.

The predefined derivative thresholds again can be determined during a calibration procedure. During the calibration procedure it is known whether a respective training pressure pulse comprises an artifact or not and, if the respective training pressure pulse does not comprise an artifact, the dicrotic notch position is known. These thresholds are determined such that the training pressure pulses with the artifacts are identified as well as possible and, for each respective training pressure pulse without artifact, the respective determined dicrotic notch position corresponds as well as possible to the respective known dicrotic notch position. It has been found that a first predefined derivative threshold within a range from 0.003 to 0.070, particularly having a value of 0.015, and a second predefined derivative threshold within a range from 0.05 to 1.00, particularly having a value of 0.25, provide good results, wherein these values refer to using mmHg as unit for the pressure and s as unit for the time in function (2) which will be described below.

The processor can be configured to determine the derivative function by dividing a) a second derivative function that depends on the second derivative by b) a first derivative function that depends on the first derivative. The division by the first derivative function allows to avoid an overweighting of the derivative function within position ranges in which the first derivative has relatively large negative values. By this avoidance of the overweighting, the notch determination maximum position can be determined such that it even better corresponds to the position of the dicrotic notch of the pressure pulse.

In a preferred embodiment, the second derivative function is the second derivative. In particular, the derivative function can be determined in accordance with following equation:

$$f\_2deri = (d^2P/dt^2)/(a+(dP/dt)^\wedge b) \quad , \qquad (2)$$

wherein f_2deri denotes the derivative function, $d^2P/dt^2$ denotes the second derivative, $dP/dt$ denotes the first derivative, and a and b denote predefined parameters. The parameters a and b can be predetermined during a calibration procedure in which for several training pressure pulses the respective position of the dicrotic notch is known and wherein the parameters a and b are determined such that the determined second derivative maximum positions correspond as good as possible to the known positions of the dicrotic notches. In a preferred embodiment a is within a range from 0 to 10 and b is within a range from 1 to 2. In particular, a is 1.0 and b is 1.3. Also these values refer to using mmHg as unit for the pressure and s as unit for the time in equation (2). It has been found that, by using the derivative function in accordance with equation (2), the determined notch determination maximum position even more accurately corresponds to the position of the dicrotic notch of the pressure pulse.

In an example, the first derivative is filtered by using a filter which preferentially is a low pass filter, wherein in an embodiment the filter depends on the heart rate. For instance, the processor can be configured such that in a normal heart rate range from, for example, 50 to 90 beats per minute, the first derivative is filtered several times, for instance three times, with a moving average filter, wherein the moving average filter comprises a window width of, for instance, 28 ms. If the actual heart rate is outside of this normal heart rate range, another filter might be used. The filtering is preferentially carried out for reducing noise, and the filter can also be constant, i.e. it also can be independent of the heart rate. Also the derivative function can be filtered by a constant filter or a filter which depends on the heart rate. In particular, also the derivative function can be filtered several times, for instance three times, with a moving average filter, wherein the moving average filter comprises a window width of, for instance, 28 ms, if the heart rate is in a normal heart rate range, for example, from 50 to 90 beats per minute, and with another filter, if the heart rate is outside this normal heart rate range. Also the filtering of the derivative function is preferentially carried out for noise reduction.

The processor further can be configured to align the derivative function with the pressure pulse. A misalignment might occur due to a filter delay, wherein this delay can be corrected by aligning the derivative function with the pressure pulse.

In an example, the processor is configured to provide the position determination function such that it provides a relation between the notch determination function position and at least one of the following characteristics of the pressure pulse: the maximum value of the pressure pulse, the maximum position along the pressure pulse, a first position along the pressure pulse before the maximum position and at which the pressure pulse has a value being a predefined first fraction of the maximum value, a second position along the pressure pulse behind the maximum position and at which the pressure pulse has a value being a predefined second fraction of the maximum value, the maximum value of the first derivative of the pressure pulse, and the difference between the pressure pulse's maximum systolic pressure and the pressure pulse's pressure at the end-diastolic point. In particular, the notch determination function position can be determined in accordance with following equation:

$$fp = c*t.dia.pre + d*t.max + e*t.dia.post + f*Pulse.max + g*d(Pulse)/dt.max + h*PP + i*PR \quad , \tag{3}$$

wherein fp denotes the notch determination function position, t.dia.pre denotes the first position along the pressure pulse before the maximum position and at which the pressure pulse has a value being a predefined first fraction of the maximum value, t.max denotes the maximum position along the pressure pulse, t.dia.post denotes the second position along the pressure pulse behind the maximum position and at which the pressure pulse has a value being a predefined second fraction of the maximum value, Pulse.max denotes the maximum value of the pressure pulse, d(Pulse)/dt.max denotes the maximum value of the first derivative of the pressure pulse, PP denotes the difference between the pressure pulse's maximum systolic pressure and the pressure pulse's pressure at the end-diastolic point and PR denotes the pulse rate. Instead of the pulse rate, also the pulse duration could be used in equation (3). It has been found that, if the notch determination function position fp is determined in this way, it can very accurately correspond to the position of the dicrotic notch of the pressure pulse.

Also the parameters c to i are predefined parameters which can be predetermined by calibration. In particular, the parameters c to i used in equation (3) can be predetermined during a calibration procedure, during which for several training pulses the position of the dicrotic notch is known and the parameters c to i are determined such that the respective notch determination function position corresponds as good as possible to the respective position of the dicrotic notch. Also the predefined first fraction of the maximum value and the predefined second fraction of the maximum value can be determined in this way, i.e. these fractions can be determined such that during the calibration procedure the resulting function position corresponds as good as possible to the known dicrotic notch positions. The predefined first and second fractions preferentially are within a range from 70 % to 95 %.

In an embodiment, the processor is configured to determine the dicrotic notch position only within a provided search range. In particular, the above described determination procedures for determining the notch determination intersection position, the notch determination maximum position and/or the notch determination function position are applied only within the search range. In an example, the search range can be predetermined based on statistical analysis. In particular, for a large group of training pressure pulses having known dicrotic notch positions an average position and a standard deviation of the dicrotic notch position can be determined and used for determining the search range. For instance, the center of the search range can be defined by the average and the width of the search range can be defined by the standard deviation.

Preferentially, the processor is configured to provide the search range such that it is defined a) by first and second percentages of the difference PP between the pressure pulse's maximum systolic pressure and the pressure pulse's pressure at the end-diastolic point and/or b) by first and second percentages of the pulse duration. For instance, start and stop points of the search range can be defined based on a) the first and second percentages of the difference between the pressure pulse's maximum systolic pressure and the pressure pulse's pressure at the end-diastolic point and/or b) the first and second percentages of the pulse duration. If several start points and/or several stop points are determined, the search range is defined by the latest start point and/or the earliest stop point, respectively. One or several of the percentages can be static, i.e. have a predefined constant value, and/or or one or several of the percentages can be dynamic, i.e. depend on the respective pulse or pulse rate. For example, start points can be defined by a static first percentage of the pulse duration, for instance, 18 % of the pulse duration, and by a static first percentage of the difference between the pressure pulse's maximum systolic pressure and the pressure pulse's pressure at the end-diastolic point, for instance, 75 % of the difference. Stop points can be defined by a static second percentage of the pulse duration, for instance, 65 % of the pulse duration, by a static second percentage of the difference between the pressure pulse's maximum systolic pressure and the pressure pulse's pressure at the end-diastolic point, for instance, 9 % of this difference, and by a dynamic percentage of the pulse duration, wherein the dynamic percentage can depend on the pulse rate.

Also these percentages are predefined by calibration, i.e. during a training phase, by analyzing training pressure pulses with known dicrotic notches. The percentages can be determined such that a sufficient appearance of the dicrotic notch is assured such that it really can be reliably detected. During the calibration procedure, for many training pressure pulses it is determined whether the dicrotic notch appears such that its position can be determined, for instance, this determination can be carried out manually by an experienced physician or technician. The value range then is determined during this calibration procedure by determining in which range relative to the respective difference PP the training pulses, which have a detectable dicrotic notch appearance, are located. It has been found that a static first percentage of the difference between the pressure pulse's maximum systolic pressure and the pressure pulse's pressure at the end-diastolic point within the range from 75 % to 85 % and a static second percentage of this difference from 5 % to 15 % allows for a further improved dicrotic notch detection. Searching for the dicrotic notch position only in the search range can be implemented by, for instance, determining the dicrotic notch only, if the pressure pulse has a value at the position, at which the first derivative of the pressure pulse has its minimum, being inside of the search range. This can ensure that the processor does not determine unreliable alleged dicrotic notch positions for pressure pulses for which a dicrotic notch position in fact is not determinable. This further increases the reliability of determining the dicrotic notch position and finally of the respiration rate based on the dicrotic notch position.

If the pressure pulse has been measured non-invasively, preferentially the processor is configured to determine the dicrotic notch position by determining the notch determination function position. It has been found that, especially in case of non-invasively measured pressure pulses, the notch determination function position corresponds very well to the dicrotic notch position.

Moreover, in a preferred embodiment, if the pressure pulse has been measured non-invasively on the skin of the encased part of the subject by using the pressure sensor while the pressure applicator increases or decreases the applied pressure, preferentially only pressure pulses are used for determining the dicrotic notch positions, which have been measured while the mean measured pressure, which could also be regarded as being the mean tissue pressure because of being measured on the skin of the tissue of the subject, is below the systolic arterial blood pressure (SAP) and above a predefined percentage of the diastolic arterial blood pressure (DAP). Preferentially, this predefined percentage is within a range from 85 % to 95 % and it is especially preferred that this predefined percentage is 90 %. The SAP and DAP values here can be values of a previous blood pressure measurement.

The processor can be configured to determine an area ratio TPA1.top/TPA2.top which is formed based on the partial areas TPA1.top and TPA2.top below a pressure pulse curve. For determining the partial areas TPA1.top and TPA2.top, a partial area TPA.top below the respective pressure pulse, i.e. below the respective pressure pulse curve, is formed, which is the area enclosed by the respective pressure pulse curve above predefined percentage of the difference TPP between the maximum and the minimum of the respective pressure pulse curve. This corresponds to the area of the respective pressure pulse curve above a horizontal line which is arranged along the predefined percentage of the difference TPP. This predefined percentage preferentially is with the range from 30 % to 70 % and further preferred it is 50 %. Then, a vertical line is arranged such that it traverses the vertex, i.e. the maximum, of the respective pressure pulse curve. Moreover, two straight lines are added, wherein a first one of the two straight lines connects the vertex with a first one of the intersections of the horizontal line with the respective pressure pulse curve and a second one of the two straight lines connects the vertex with a second one of the intersections of the horizontal line with the respective pressure pulse curve. The first straight line together with the horizontal line and the vertical line enclose the first partial area TPA1.top and the second straight line together with the horizontal line and the vertical line enclose the second partial area TPA2.top. These partial areas TPA1.top and TPA2.top are exemplarily visualized also in Fig. 6A and described from page 19, line 36 to page 20, line 8 of WO 2018/210931 A1 which is herewith incorporated by reference.

In an embodiment the processor is configured to determine a moving average of the area ratio TPA1.top/TPA2.top over a predetermined number of pressure pulses. Then, for each respective pressure pulse, a difference between the moving average of the area ratio TPA1.top/TPA2.top and the respective area ratio TPA1.top/TPA2.top of the respective pressure pulse is calculated. Based on the resulting differences, a standard deviation function is calculated for the pressure pulses. A corresponding standard deviation function (TPA1.top/TPA2.top).sd is exemplarily visualized in Fig. 6B and described on page 9, lines 19 to 26 and on page 20, lines 10 to 23 of WO 2018/210931 A1 which, as mentioned above, is incorporated by reference. The processor can be configured to use only pressure pulses for determining the dicrotic notch positions, which have been measured before the standard deviation function reached its maximum.

In an example, the processor is configured to, for a respective pressure pulse, determine, as the at least one additional feature that characterizes the respective pressure pulse and that does not necessarily depend on the dicrotic notch position, preferentially at least one of the following features: i) the difference between the pressure pulse's maximum systolic pressure and the pressure pulse's pressure at the end-diastolic point, ii) the area enclosed by an upper part of the pressure pulse, wherein a) the upper end of the upper part is at the pressure pulse's maximum systolic pressure and b) the lower end of the upper part is between the pressure pulse's maximum systolic pressure and a pressure value which corresponds to a mean of the pressure, iii) the duration of the respective pressure pulse, iv) the area of the respective pressure pulse, and v) the width at half maximum of the respective pressure pulse. It has been found that by using at least one of these additional features the determination of the respiration rate can be further improved.

The difference between the pressure pulse's maximum systolic pressure and the pressure pulse's pressure at the end-diastolic point preferentially is the difference between the maximum measured pressure and the minimum measured pressure of the respective pressure pulse. The duration of the respective pressure pulse preferentially corresponds to the time difference between an end-diastolic point and the following end-diastolic point for the respective pressure pulse. The width at half maximum corresponds to the width at 50 percent of the difference between the maximum pressure and the minimum pressure of the respective pressure pulse. Thus, it is the width at 50 percent of the difference between the pressure of the pressure pulse at the maximum systolic point and the pressure of the pressure pulse at an end diastolic point.

In another aspect of the present invention a method for determining a respiration rate of a subject is presented, wherein the method comprises:

- providing a pressure signal of a subject over a time by a pressure signal providing unit, wherein the pressure signal is indicative of blood pulsations and comprises a plurality of pressure pulses,
- determining, for a respective pressure pulse of the plurality of pressure pulses, a respective dicrotic notch position by a processor,
- determining the respiration rate based on the dicrotic notch positions determined for the plurality of pressure pulses.

In an aspect of the present invention a computer program for determining a respiration rate of a subject is presented, wherein the computer program comprises program code means for causing the apparatus for determining a respiration rate as defined by claim 1 to carry out the steps of the method as defined by claim 14.

The apparatus, method and computer program for determining the respiration rate can be adapted to determine the respiration rate continuously, i.e. several subsequent respiration rate measurements are carried out, or to determine the respiration rate non-continuously, i.e. for instance one time.

It shall be understood that the apparatus of claim 1, the method of claim 14, and the computer programs of claim 15, have similar and/or identical preferred embodiments, in particular, as defined in the dependent claims.

It shall be understood that a preferred embodiment of the present invention can also be any combination of the dependent claims or above embodiments with the respective independent claim. These and other aspects of the invention will be apparent from and elucidated with reference to the embodiments described hereinafter.

BRIEF DESCRIPTION OF THE DRAWINGS

In the following drawings:

Fig. 1 shows schematically and exemplary an embodiment of an apparatus for determining a respiration rate of a subject, which comprises a measurement device, in a situation in which a pressure cuff of the measurement device is inflated,
Fig. 2 shows schematically and exemplary a shell of the measurement device encasing an upper arm of the subject,
Fig. 3 shows schematically and exemplary the apparatus in a situation in which the pressure cuff is deflated,
Fig. 4 shows schematically and exemplary a measured tissue pressure and further values derived from the tissue pressure measurement,
Fig. 5 illustrates schematically and exemplary a determination of a dicrotic notch position being an intersection position of a shifted tangent and a respective pressure pulse,
Fig. 6 illustrates schematically and exemplary a determination of a dicrotic notch position being a second derivative maximum position, i.e. a position at which the second derivative of a respective pressure pulse has a maximum,
Fig. 7 illustrates schematically and exemplary a determination of a search range,
Fig. 8 illustrates schematically and exemplary a determination of a dicrotic notch position being a function position,
Fig. 9 illustrates calculations of different features for a pressure pulse of the measured tissue pressure, which depend on the determined dicrotic notch position,
Figs. 10 to 13 illustrate calculations of different additional features for a pressure pulse of the measured tissue pressure, and
Fig. 14 shows a flowchart exemplary illustrating an embodiment of a method for determining a respiration rate of a subject.

DETAILED DESCRIPTION OF EMBODIMENTS

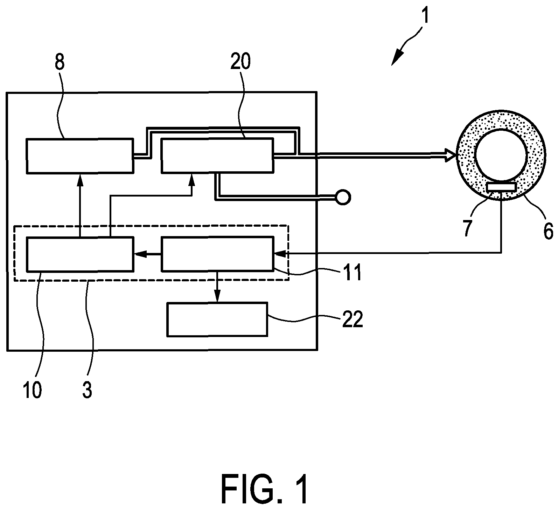

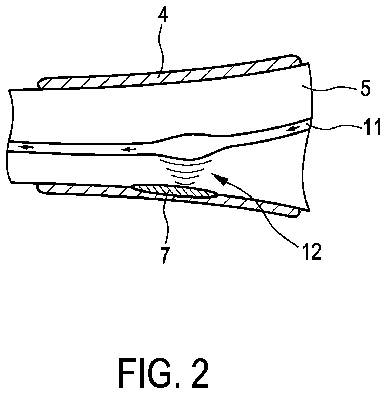

Fig. 1 shows schematically and exemplarily an apparatus 1 for determining a respiration rate of a subject. The apparatus 1 comprises a shell 4 which can be seen in Fig. 2 and which is configured to encase a part 5 of the subject, through which blood flows. In this embodiment the part 5 of the subject is an arm of the subject, wherein in Fig. 2 within the arm 5 a brachial artery 11 is shown and wherein the arrows within the brachial artery 11 indicate the flow direction of the blood away from the heart. The apparatus 1 further comprises a pressure sensor 7 arranged inside the shell 4 and configured to measure the pressure on the outer skin of the encased arm 5 of the subject. The measured pressure can also be regarded as being tissue pressure (TP). As indicated in Fig. 2, a pulse wave within the brachial artery 11 causes a pressure wave 12 which is transmitted to the pressure sensor 7 via the tissue of the arm 5. The shell 4 is not shown in Fig. 1 for clarity reasons.

The apparatus 1 further comprises a cuff 6 which encloses the shell 4 and which is inflatable by using a pump 8 for applying, from outside the shell 4, pressure to the shell 4 and thereby to the encased arm 5 of the subject. Since the cuff 6 and pump 8 together enable an application of the pressure to the shell 4 and thereby to the encased arm 5 of the subject, they can be regarded as forming a pressure applicator 6, 8. Moreover, the shell 4, the pressure applicator 6, 8 and the pressure sensor 7 can be regarded as being components of a measurement device which is controlled by a processor 3. The shell 4 with the cuff 6 is preferentially a kinking-proof shell cuff as described in WO 2014/121945 A1.

The processor 3 is configured to control the measurement device such that the pressure applicator 6, 8 increases the applied pressure in a measurement time period extending till an end measurement time point and decreases the applied pressure in a following post-respiration-rate-measurement time period and that the pressure sensor 7 measures the pressure on the skin, i.e. the tissue pressure TP, at least during the measurement time period. Moreover, the processor 3 is configured to control the pressure applicator 6, 8 such that it increases the applied pressure with a first rate in a pre-measurement time period which is followed by the measurement time period in which the applied pressure is increased with a second rate, wherein the first rate is larger than the second rate. The control of the measurement device such that the cuff 6 is inflated and hence the applied pressure is increased is illustrated in Fig. 1, i.e. the bold arrows indicate the inflation situation.

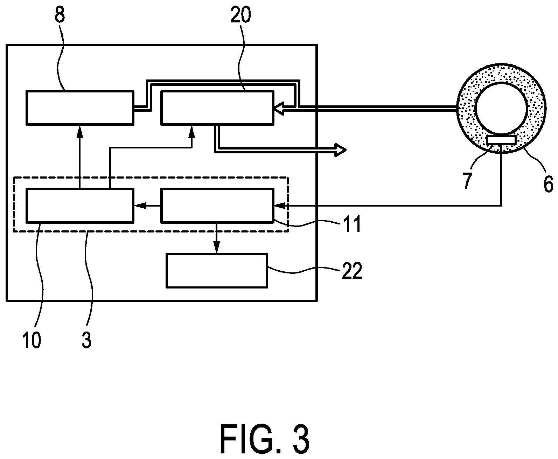

The apparatus 1 comprises a valve 20 which, when the valve 20 is opened, allows the compressed air within the system to leave the system into the surrounding atmosphere for deflating the cuff. In Fig. 3 this deflation situation, in which the pump 8 is switched off, is indicated by the bold arrows.

The processor 3 can be regarded as comprising a controlling part 10 for controlling the pump 8 and the valve 20 and a processing part 11, which especially is configured to carry out some calculations that will be explained further below. The apparatus 1 can also comprise a display 22 for displaying the determined respiration rate and optionally further physiological parameters like the blood pressure.

In the pre-measurement time period, which could also be regarded as being a fast inflation period, the processor 3 controls the apparatus 1 such that the valve 20 is closed and the pump 8 inflates the cuff 6 with the larger first rate. In the following measurement time period the processor 3 also controls the apparatus 1 such that the valve 20 is closed, but the pump 8 is controlled such that the inflation of the cuff 6 is continued with the lower second rate. The measurement time period could therefore also be regarded as being a slow inflation period.

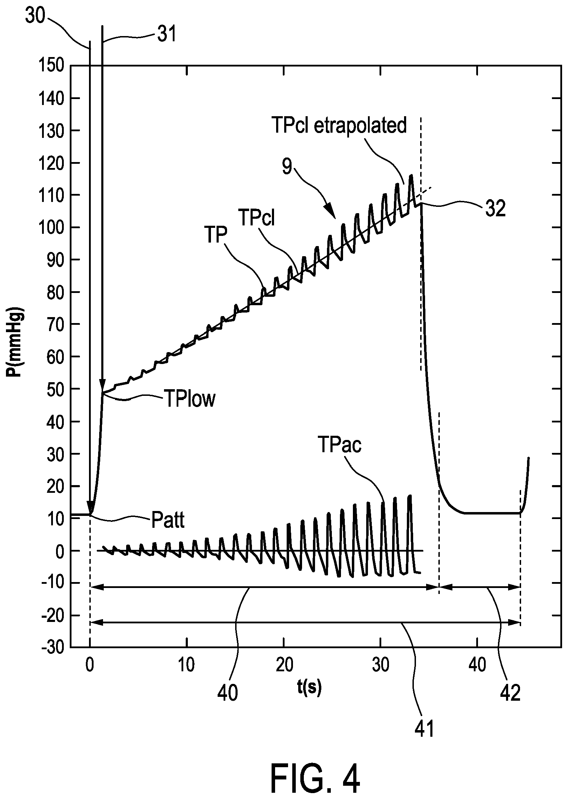

Fig. 4 schematically and exemplarily illustrates the measured pressure TP versus time t. The first inflation in the pre-measurement time period starts with a tissue pressure TP being an attachment pressure Patt that is the measured tissue pressure in the uninflated cuff 6. This attachment pressure Patt can range, for instance, from 0 to 15 mmHg. An attachment pressure Patt up to 15 mmHg has turned out to not result in venous congestion for more than 12 hours, thereby making the assembly of the shell 4 with the cuff 6 optimally suitable for longer term monitoring. For this reason, it is preferred that the attachment pressure Patt is not larger than 15 mmHg. In Fig. 4 the arrow 30 indicates the start of the pre-measurement time period, i.e. the start of the fast inflation period. During this pre-measurement time period the part of the overall tissue pressure range, which has no or substantially no information for the determination of the blood pressure, shall be passed as fast as possible. Therefore, the inflation rate is preferentially as large as possible during the pre-measurement time period. For instance, the inflation rate with respect to the tissue pressure TP can be equal to or larger than 8 mmHg/s. This pre-measurement time period with a fast inflation rate ends at a tissue pressure value which is indicated in Fig. 4 by "TPlow".

The tissue pressure value TPlow also indicates the start of the measurement time period with the slower second inflation rate. In Fig. 4 this start of the measurement time period, which can also be regarded as being a slow inflation period, is indicated by the arrow 31.

In Fig. 4 the time interval 40 indicates the inflation-deflation time period from the start 30 of fast inflation until the tissue pressure TP has dropped below 20 mmHg during the fast deflation, in order to allow venous return. The time period 41 indicates a cycle time being the time between a start of a measurement and a start of a following measurement and the time period 42 indicates a break period being the difference between the inflation-deflation time period 40 and the cycle time 41.

Fig. 4 also illustrates mean pressure TPcl that can be regarded as being a tissue clamping pressure affecting the tissue when the cuff 6 is attached to the arm 5, for instance, to the upper arm, of the subject. The mean pressure TPcl can be calculated by applying a low pass filter to the tissue pressure TP, wherein the low pass filter might be located within the processor 3. The processor 3 is preferentially configured to determine an alternating component TPac of the tissue pressure by subtracting the mean pressure TPcl from the measured tissue pressure TP, i.e. TPac = TP - TPcl. In Fig. 4 the TPac curve is shown enlarged by a factor of 2, in order to improve visibility of this curve.

During the slow inflation period, i.e. during the measurement time period, TP pulse curves and/or TPac pulse curves are recorded, which may be analyzed simultaneously, i.e. online, wherein the TP pulse curves and/or the TPac pulse curves have tissue pressure waveforms (TPW) comprising information which allows for an accurate determination of the respiration rate.

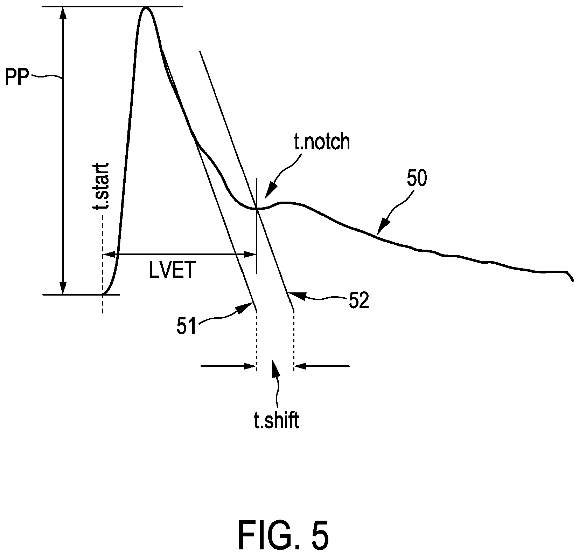

An important characteristic of the pressure pulses, i.e. of the TP pulse curves and of the TPac pulse curves, is the position of the dicrotic notch. As it will be explained in the following with reference to Figs. 5, 6 and 7, the processor 3 therefore is configured to determine the position of the dicrotic notch. For instance, the processor can be configured to determine the dicrotic notch position by determining at least one of a) a notch determination intersection position being a position at which a shifted tangent 52 intersects with the pressure pulse, wherein the shifted tangent 52 is determined by determining a tangent 51 to the pressure pulse at a position of the pressure pulse, at which the first derivative of the pressure pulse has a minimum, and by shifting the tangent 51 in the direction of increasing time by a shifting distance t.shift, b) a notch determination maximum position being a position at which a derivative function f_2deri has a maximum, wherein the derivative function is determined by determining the first derivative of the pressure pulse, by determining the second derivative of the pressure pulse and by combining the determined first and second derivatives, and c) a notch determination function position that is determined by determining a characteristic of the pressure pulse and by applying a position determination function to the characteristic, wherein the position determination function provides a relation between the characteristic and the notch determination function position.

The processor 3 can be configured to determine one, two or all of the notch determination intersection position, the notch determination maximum position and the notch determination function position. If the processor 3 determines two or three of the notch determination intersection position, the notch determination maximum position and the notch determination function position, the processor can be configured to combine the different positions to obtain a combined position that corresponds to the dicrotic notch position. For instance, the different positions can be averaged for determining the dicrotic notch position.

Before determining, for instance, the notch determination intersection position, the processor 3 preferentially checks whether the pressure pulse has a value at the position, at which the first derivative of the pressure pulse has its minimum, being within a search range defined by first and second percentages of the difference PP between the pressure pulse's maximum systolic pressure and the pressure pulse's pressure at the end-diastolic point. This search range also could be named first search range. Preferentially, the static first percentage of the difference PP is within the range from 75 % to 85 % and a static second percentage of this difference PP is within the range from 5 % to 15 %. For instance, this search range can range from 75 % of PP to 10 % of PP.

If the pressure pulse has a value at the position, at which the first derivative of the pressure pulse has its minimum, being within the predefined search range that depends on the difference PP, the processor 3 determines the tangent 51 at the position along the respective pulse 50 at which the first derivative has its minimum. As illustrated in Fig. 5, this tangent 51 is shifted by the shifting distance t.shift, wherein the intersection position of the shifted tangent 52 and the pulse 50 corresponds to the dicrotic notch position t.notch. In Fig. 5, this shift is a right shift, because the increasing time direction is from left to right in this Fig. If several intersection points of the shifted tangent 52 with the pressure pulse exist, the processor 3 preferentially chooses the temporally last intersection position as the intersection position to be used for determining the dicrotic notch position.

The processor 3 can be configured to provide the shifting distance t.shift such that it is dependent on a characteristic of the respective pressure pulse 50. The characteristic can be any characteristic that is related to the pressure pulse and the shifting distance can depend on one or several characteristics. For instance, the shifting distance can depend on at least one of a) the pulse rate (PR), which could also be named heart rate, b) the position of the minimum of the first derivative of the pressure pulse in between the pressure pulse's maximum systolic pressure and the pressure pulse's pressure at the end-diastolic point and/or in between the time of the pressure pulse's maximum systolic pressure and the time at the end of the pressure pulse, c) the minimum value of the first derivative and d) the width of the pressure pulse at a predefined percentage of the difference PP. The predefined percentage preferentially is within a range from 50 % to 80 %. In a preferred embodiment the predefined percentage is 66 %. In an example, the width of the pressure pulse at the predefined percentage of the difference between the pressure pulse's maximum systolic pressure and the pressure pulse's pressure at the end-diastolic point is divided by the full pulse length, i.e. by the pulse duration, in order to determine the shifting distance. However, the processor 3 can also be configured to provide a constant or static shifting distance t.shift, i.e., for instance, a shifting distance t.shift being independent of any characteristic of the pressure pulse 50.

Fig. 5 further shows the position t.start at which the pressure pulse 50 starts and the difference LVET (left ventricular ejection time) between the start position t.start and the determined intersection position which corresponds to the dicrotic notch position t.notch.

Also before determining the notch determination maximum position, the processor 3 preferentially checks whether the pressure pulse has a value at the position, at which the first derivative of the pressure pulse has its minimum, being within the above mentioned search range defined by percentages of the difference PP between the pressure pulse's maximum systolic pressure and the pressure pulse's pressure at the end-diastolic point. If this is the case, the notch determination maximum position will be determined. Otherwise, the notch determination maximum position will not be determined for the pressure pulse and it will be proceeded with the next pressure pulse of the pressure signal.

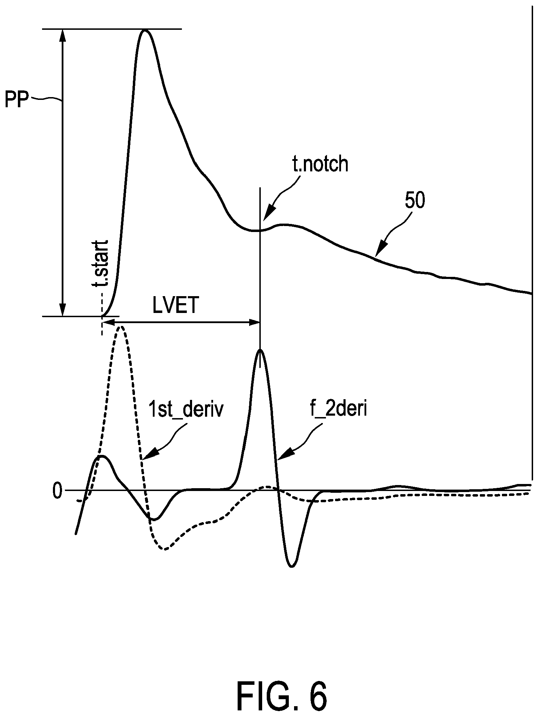

For determining the notch determination maximum position, the processor 3 can be configured to calculate a position at which a derivative function has a maximum, wherein the derivative function is determined by determining the first derivative of the pressure pulse, by determining the second derivative of the pressure pulse and by combining the determined first and second derivatives. The first derivative can be filtered, in order to reduce noise. In particular, depending on in which heart rate range the actual heart rate is located, different noise reduction filters can be used. In an embodiment, if the heart rate is within a normal heart range from 50 to 90 beats per minute, the filter can be a moving average filter, wherein, for example, a filter having a width of 28 ms or having another width can be applied several times, for example, three times. The filtered first derivative 1st_deriv is exemplarily illustrated in Fig. 6.

The processor 3 can be further configured to determine the derivative function by dividing a) a second derivative function that depends on the second derivative by b) a first derivative function that depends on the first derivative. Preferentially, the second derivative function is the second derivative and the derivative function is determined in accordance with equation (2). Also the second derivative and the derivative function can be filtered for noise reduction as explained above with respect to the first derivative. Also the filtered derivative function f_2deri is exemplarily illustrated in Fig. 6.

The processor 3 further can be configured to define a further search range in which it should be searched for maxima of the derivative function. In particular, the processor can be configured to use a predefined search range that has been predetermined by, for instance, statistical analysis as described above or that has been determined by start and stop points as it will be explained in the following. This further search range might be regarded as being a second search range.

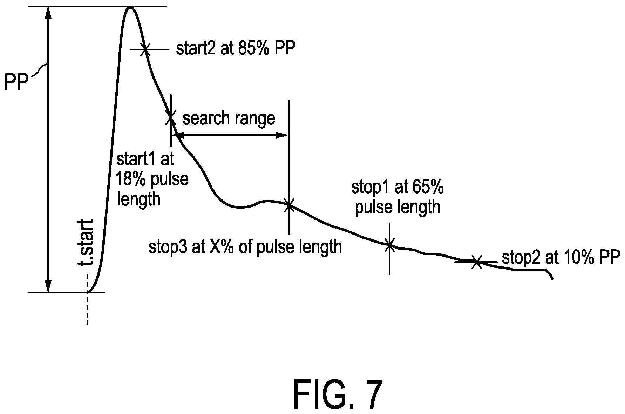

The processor 3 can be configured to determine start and stop points of the further search range based on a) percentages of the difference PP and b) percentages of the pulse duration. In particular, several start points and several stop points can be determined, wherein the further search range can be defined by the latest start point and the earliest stop point and wherein the percentages can be static or depend on the respective pulse or pulse rate. For example, as illustrated in Fig. 7, a start point start1 can be defined by a static first percentage of the pulse duration of, for instance, 18 %, a further start point start2 can be defined by a static first percentage of the difference PP, for instance, of 85 %, a first stop point stop1 can be defined by a static second percentage of the pulse duration, for instance, of 65 %, a second stop point stop2 can be defined by a static second percentage of the difference PP, for instance, of 10 % and a third stop point stop3 can be defined by a dynamic percentage of the pulse duration, which depends on the pulse rate and which can be, for example, 43 % for a pulse rate of 50 bpm. In Fig. 7 the search range starts at the latest start point start1 and ends at the earliest stop point stop 3.

Preferentially, the processor 3 is configured to determine all maxima of the derivative function f_2deri within the further search range which are larger than a first predefined derivative threshold, in order to determine all large maxima of the second derivative within this search range. If the highest maximum of the determined maxima is larger than a product of a predefined factor and the second highest maximum in this search range, the position of the highest maximum within this search range is regarded as being the notch determination maximum position, i.e. as corresponding to the dicrotic notch position t.notch. Otherwise, the position of the temporally first maximum of the derivative function f_2deri within this search range is regarded as being the notch determination maximum position, i.e. as corresponding to the dicrotic notch position t.notch.

If within the further search range two maxima are found, which are larger than a second predefined derivative threshold being larger than the first predefined derivative threshold used for determining the large maxima, i.e. if within this search range two or more very large maxima are present, the respective pressure pulse is not considered for determining the respiration rate, because it is regarded as having an artifact.

Also before determining the notch determination function position, the processor 3 can check whether the pressure pulse has a value at the position, at which the first derivative of the pressure pulse has its minimum, being within the first search range that depends on the difference PP between the pressure pulse's maximum systolic pressure and the pressure pulse's pressure at the end-diastolic point. If this is the case, the notch determination function position will be determined. Otherwise, the notch determination function position will not be determined for the pressure pulse and it will be proceeded with the next pressure pulse of the pressure signal.

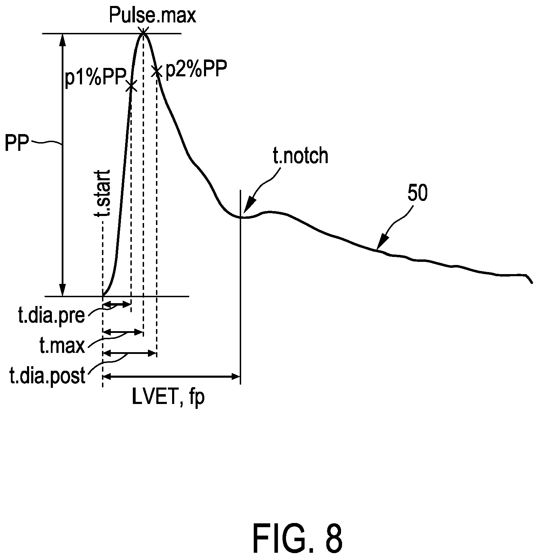

In order to determine the notch determination function position, the processor 3 can be configured to provide the position determination function such that it defines a relation between the notch determination function position and at least one of the following characteristics of the respective pressure pulse which are also partly illustrated in Fig. 8: the maximum value Pulse.max of the respective pressure pulse, the maximum position t.max along the respective pressure pulse, a first position t.dia.pre along the respective pressure pulse before the maximum position t.max and at which the respective pressure pulse has a value being a predefined first fraction p1 of the maximum value Pulse.max, a second position t.dia.post along the respective pressure pulse behind the maximum position t.max and at which the respective pressure pulse has a value being a predefined second fraction p2 of the maximum value Pulse .max, the maximum value d(Pulse)/dt.max of the first derivative of the respective pressure pulse, and the difference PP between the pressure pulse's maximum systolic pressure and the pressure pulse's pressure at the end-diastolic point. In particular, the processor 3 can be configured to calculate the notch determination function position fp, which should correspond to the dicrotic notch position t.notch, in accordance with above equation (3).

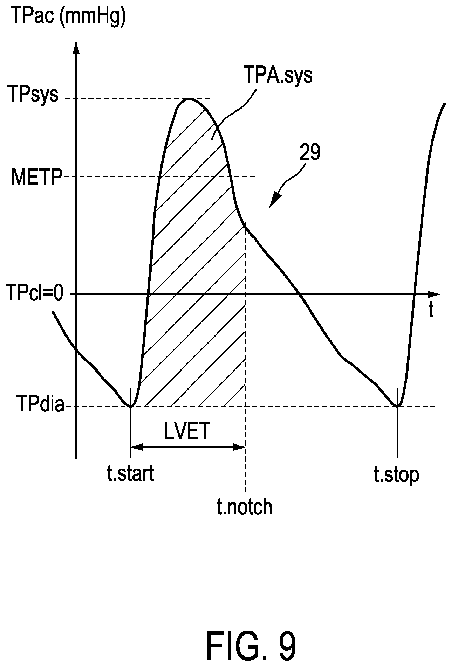

The processor 3 is configured to determine, for the respective pressure pulse, at least one respective feature, which characterizes the respective pressure pulse, based on the respective determined dicrotic notch position. In the following this will be described with reference to Fig. 9.

As can be seen in Fig. 9, the pressure pulse 29 has a notch in its falling slope, wherein the time, at which the notch occurs, is denoted by "t.notch". In particular, the pressure pulse 29, which reaches from t.start to t.stop and which thus has a pulse curve duration of t.stop - t.start, comprises a systole starting at t.start with a systolic upslope and ending at the dicrotic notch at the time t.notch in the following downslope. In this example, the dicrotic notch is characterized by a slight incision. The further decreasing section of the pulse curve from t.notch to t.end can be called "diastole".

The processor can be adapted to determine the difference between the start of the pressure pulse 29 (t.start) and the position of the notch (t.notch) as a feature to be used for determining the respiration rate or another physiological parameter. This difference, i.e. this feature, can be named "systolic time interval" or "left ventricular ejection time" (LVET).

Moreover, the processor 3 can be configured to determine the area TPA.sys under the pressure pulse 29 between the start position t.start and the notch position t.notch. The corresponding area is schematically and exemplarily indicated in Fig. 9. In an embodiment, the processor 3 can be configured to determine, additionally or alternatively, another feature that characterizes the ejection phase, in particular the left ventricular ejection phase of the pressure pulse 29, wherein this other feature also uses the knowledge about the dicrotic notch position defining the transition between the systole and the diastole.

In an embodiment, the processor 3 is further configured to determine, for the respective pressure pulse, a respiration rate determination value TPWP_R based on at least one feature, which characterizes the respective pressure pulse and which depends on the dicrotic notch position, such that for several pressure pulses, which are present at different times, several respiration rate determination values TPWP_R are determined. The several respiration rate determination values TPWP_R determined for the several pressure pulses and hence for the several times form a respiration rate determination curve. The respiration rate determination curve might also be named respiratory pulse variation signal.

In a preferred embodiment, the respiration rate determination values TPWP_R are determined based on at least one of TPA.sys and LVET. Moreover, in a preferred embodiment, the respiration rate determination value TPWP_R is identical to or linearly related to, particularly proportional to, TPA.sys. Hence, preferentially the respiration rate determination curve, which might also be named respiratory pulse variation signal, is TPA.sys(t), i.e. the respiration rate determination curve or respiratory pulse variation signal can be formed by using a sequence of TPA.sys values that have been determined over time. However, as indicated before, the respiration rate determination value TPWP_R can also be determined by combining TPA.sys and LVET, for instance, by linearly combining TPA.sys and LVET.

The processor 3 can be further configured to apply a frequency range pre-selecting filter to the respiratory pulse variation signal such that the respiration rate is retained in the respiratory pulse variation signal. This filter can be, for instance, a band pass filter that is configured such that parts of the respiratory pulse variation signal, which are above a lower cut-off frequency and below a higher cut-off frequency, pass the filter. In an example, the band pass filter is a combination of a high pass filter with a lower cut-off frequency below the lowest expected respiration rate and a low pass filter with a higher cut-off frequency above the largest expected respiration rate. For instance, the lower cut-off frequency of the band pass filter, i.e., for example, the cut-off frequency of the high pass filter, can be within the range of 0.02 Hz to 0.1 Hz. In a preferred embodiment this cut-off frequency is 0.083 Hz, wherein it is assumed that the lowest expected respiration rate is larger than 5 breaths per minute, e.g. 6 breaths per minute. The higher cut-off frequency of the band pass filter, i.e., for example, the cut-off frequency of the low pass filter, can be within the range of 0.583 Hz to 0.833 Hz. In a preferred embodiment this cut-off frequency is 0.667Hz, wherein it is assumed that the highest expected respiration rate is lower than 40 breaths per minute, e.g. 36 breaths per minute.

In this embodiment the processor 3 is configured to determine the respiration rate based on the respiratory pulse variation signal. In particular, the processor 3 is configured to transform the signal from the time domain to the frequency domain and to determine the frequency in the frequency domain. For transforming from the time domain to the frequency domain corresponding transformations can be used like a Fourier transformation, in particular a fast Fourier transformation, a wavelet transformation, et cetera. For instance, the respective frequency at the respective maximum in the frequency domain, particularly within a predefined expected frequency range, can be determined as the respective respiration rate. The predefined expected frequency range is a frequency range in which respiration rates of a subject are to be expected.

The processor 3 also can be configured to determine additional features for the respective pressure pulse. In the following a determination of additional features for a respective processed measured pressure pulse 29 will be described, wherein in this example the respective processed measured pressure pulse 29 is a respective TPac pulse curve.

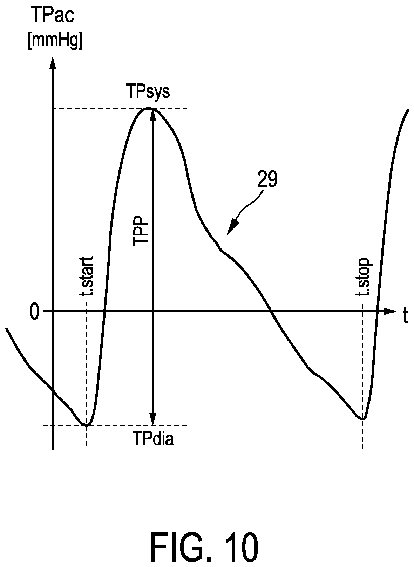

For instance, the difference TPP between the maximum measured pressure and the minimum measured pressure, i.e. the difference of the pressure pulse's maximum systolic pressure (TPsys) and the pressure pulse's pressure at an end-diastolic point (TPdia), as indicated in Fig. 10, can be determined.

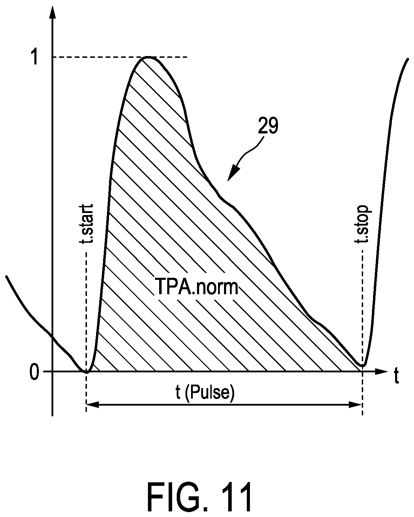

The processor 3 can also be configured to determine the pulse duration (t(Pulse)) being the time difference between an end-diastolic point and a following end-diastolic point of the pressure pulse 29. This feature can also be defined as being the temporal difference between the start of the respective pulse (t.start) and the end of the respective pulse (t.stop). This feature is indicated in Fig. 11.

The processor 3 can also be adapted to determine the pulse area (TPA) of the respective pulse 29 being the area under the respective pulse curve within the time defined by t.start to t.stop and ranging from the pressure pulse's pressure at an end-diastolic point TPdia to a pressure pulse's maximum systolic pressure TPsys. Preferentially, the pulse area TPA is scaled to TPP=1 as indicated in Fig. 11. This scaled pressure pulse area is named "TPA.norm".

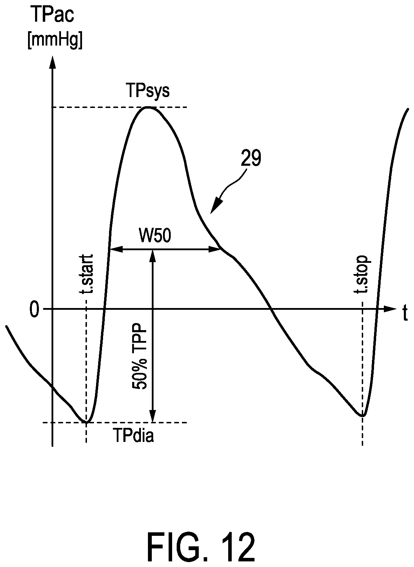

The processor 3 can also be adapted to determine the pulse width at half maximum (W50) of the respective pulse 29 as indicated in Fig. 12.

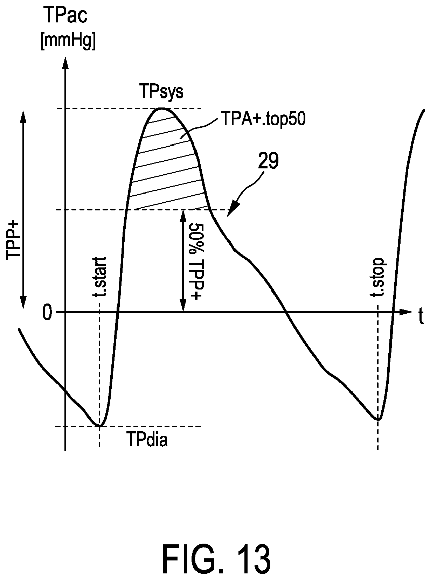

Moreover, the processor 3 can be adapted to determine the area TPA+.top50 enclosed by an upper part of the pressure pulse 29, as schematically illustrated in Fig. 13. The upper end of the area TPA+.top50 enclosed by the upper part is at the pressure pulse's 29 maximum systolic pressure TPsys and the lower end of the area TPA+.top50 of the upper part is between the pressure pulse's 29 maximum systolic pressure TPsys and a pressure value which corresponds to the mean TPcl of the measured pressure TP. Since the pressure pulse 29 has been processed by subtracting the mean TPcl from the measured pressure TP, the pressure value, which corresponds to the mean TPcl of the measured pressure TP, is zero. Preferentially, the processor 3 is configured to determine the area TPA+.top50 enclosed by the upper part of the pressure pulse such that the lower end of the upper part is at a pressure value which corresponds to half of the pressure distance TPP+ between the pressure pulse's maximum systolic pressure TPsys and the pressure value which corresponds to the mean TPcl of the measured pressure TP.

In Figs. 9 to 13 reference signs are used, which have as a first letter a "T", because in this example the measured pressure signal is a tissue pressure signal. However, as explained above, instead of a tissue pressure signal, also another kind of pressure signal can be measured, which is indicative of blood pulsations and comprises a plurality of pressure pulses. For instance, a pressure signal can be invasively measured and the above described determination of the dicrotic notch position and the physiological parameter can be applied to the pressure pulses of the invasively measured pressure signal.

The processor 3 further can be configured to determine for a respective pressure pulse a blood pressure determination value TPWP_M based on at least one determined feature such that for several pressure pulses, which are present at different times, several blood pressure determination values TPWP_M are determined, wherein the several blood pressure determination values TPWP_M determined for the several pressure pulses and hence for several times form a blood pressure determination curve TPW_M-curve. The processor 3 can be configured to determine the blood pressure based on the blood pressure determination curve TPW_M-curve, for instance as explained in WO 2018/210931 A1. In particular, the processor 3 can be configured to determine a position of a maximum (TPW_M-curve.max) of the blood pressure determination curve TPW_M-curve and to determine the blood pressure based on the determined position and the measured pressure TP. For instance, the processor 3 can be configured to determine the systolic arterial blood pressure (SAPni) based on the mean TPcl of the measured pressure TP at the determined position of the maximum (TPW_M-curve.max) in accordance with following equation:

$$SAPni = \alpha \cdot (TPcl@TPW\_M\text{-curve.max}) \quad , \quad (4)$$

wherein the parameter $\alpha$ can be predetermined by calibration.

In fact, the processor 3 can be configured to determine a position of the maximum of the blood pressure determination curve TPW_M-curve and/or a position of a derivative, such as the first derivative, for instance, of the blood pressure determination curve TPW_M-curve, and to determine the blood pressure based on one or both of the determined positions and the measured pressure TP. For instance, the processor 3 can be configured to determine the systolic arterial blood pressure based on the mean TPcl of the measured pressure TP at one or both of the determined positions in accordance with SAPni = $\alpha$' · (TPcl@TPW_M-curve.max) + $\beta$' · (TPcl@TPW_M-curve'.max), wherein TPW_M-curve' refers to the derivative of TPW_M-curve, and wherein the parameters $\alpha$' and $\beta$' can be predetermined by calibration.

In an embodiment the respiration rate determination curve and/or the blood pressure determination curve is a smoothed curve, wherein a smoothing procedure used for smoothing the respective physiological parameter determination curve can include, for instance, filtering and/or fitting. For smoothing the respective physiological parameter determination curve a moving average filter, particularly a variable moving average filter, can be used that is applied on the physiological parameter determination values, i.e. in this case the respiration rate determination values and blood pressure determination values, determined for the pressure pulses. The window used for the averaging can be fixed or variable, wherein in the latter case it preferentially has a maximum duration of, for instance, 8 s. The use of the filter can cause a filter delay, wherein the minimum filter delay is as long as the added up durations of the several pulses.

The measurement of the physiological parameter, i.e., for instance, of the respiration rate and the blood pressure, is intended to be used in a sequence of measurements in rapid succession to allow for effective semi-continuous monitoring such that the stress for the monitored individual, i.e. for the monitored subject, is minimized.

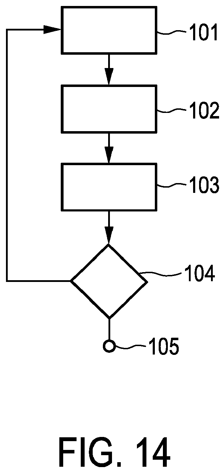

In the following an embodiment of a method for determining a respiration rate of a subject will be exemplary described with reference with to a flowchart shown in Fig. 14.

In step 101, a measured pressure signal of the subject is provided over time by the pressure signal providing unit, wherein in this embodiment the pressure signal has been measured by using a measurement device comprising a) a shell configured to encase a part of the subject, through which blood flows, b) a pressure applicator configured to apply pressure to the shell and thereby to the encased part of the subject, and c) a pressure sensor configured to measure the pressure signal on the skin of the encased part of the subject, wherein the pressure applicator increases or decreases the applied pressure while the pressure signal is measured, and wherein the measured pressure signal is indicative of blood pulsations and comprises a plurality of pressure pulses. In particular, the pressure signal is measured by using the measurement device described above with reference to Figs. 1 to 3.

In step 102, for a respective pressure pulse of the plurality of pressure pulses, a respective feature that characterizes the respective pressure pulse is determined thereby determining a plurality of features for the plurality of pressure pulses. Moreover, in step 102, the respiration rate is determined based on the determined plurality of features. For each pressure pulse, one or several features, i.e. one or several different types of features, can be determined. The one or several features, which are determined for a respective single pressure pulse, include at least one feature that depends on the dicrotic notch position.

The dicrotic notch position can be determined based on at least one of the notch determination intersection position, the notch determination maximum position and the notch determination function position, which can be determined for the respective pressure pulse. In particular, the notch determination intersection position can be determined as the intersection of a shifted tangent on the respective pressure pulse, wherein the shifted tangent is determined by determining a tangent to the respective pressure pulse at a position of the respective pressure pulse, at which the first derivative of the respective pressure pulse has a minimum, and by shifting the tangent in the direction of increasing time by a shifting distance. The notch determination maximum position can be determined as a position at which the derivative function of the respective pressure pulse has a maximum, wherein for more details in this regard reference is made to the corresponding description provided above. Finally, the notch determination function position can be determined by determining a characteristic of the respective pressure pulse and by applying a position determination function to the characteristic, wherein the position determination function provides a relation between the characteristic and the notch determination function position. For instance, equation (3) can be used for determining a notch determination function position for a respective pressure pulse.

The notch determination intersection position or the notch determination maximum position or the notch determination function position can directly be the dicrotic notch position to be determined, or at least one of the notch determination intersection position, the notch determination maximum position and the notch determination function position can be combined, especially averaged, for determining the dicrotic notch position. Moreover, the dicrotic notch position determined for the respective pressure pulse can directly be the feature or it can be processed for providing the respective feature. For instance, as explained above with respect to Fig. 9, the determined dicrotic notch position t.notch can be used for determining the feature LVET and/or the feature TPA.sys.

In step 103, the features determined for the several pressure pulses are used for determining respiration rate determination values TPWP_R which form a respiration rate determination curve or respiratory pulse variation signal, wherein the respiration rate is determined based on the respiratory pulse variation signal, as described above.

In step 104, it is determined whether an abort criterion is fulfilled. If this is the case, the method stops in step 105, otherwise it proceeds with step 101. Thus, the method can be carried out in a loop for continuously monitoring the respiration rate and optionally one or more further physiological parameters like the blood pressure over time in several measurement cycles, until the abort criterion is fulfilled. For instance, the monitoring can be interrupted, if a user like a physician has input a corresponding command into the apparatus via an input unit like a keyboard, a computer mouse, a touchpad, et cetera.

Although in above described embodiments features are determined by using the TPac pulses as processed pressure pulses, the features can also be determined by using directly the measured pressure pulses, i.e., for instance, the TP pulses. It is also possible to process the pressure pulses in another way, i.e., for example, not by subtracting the mean TP value for determining the TPac pulses. For instance, for the respective measured pressure pulse the pressure values at t.start and t.stop can be connected by a straight line and this straight line can be subtracted from the respective measured pressure pulse, in order to determine a processed pressure pulse.

Although in above described embodiments the pressure signal is a tissue pressure signal that has been measured by using a shell cuff, i.e. although in above described embodiments the pressure signal has been measured non-invasively, it is also possible that the pressure signal is measured invasively. For measuring the pressure signal invasively, known corresponding measurement devices can be used like the measurement device described in the article "How to measure blood pressure using an arterial catheter: a systematic 5-step approach" by B. Saugel et al., Critical Care, 24:172 (2020), which is herewith incorporated by reference. Also other known techniques can be used for measuring the pressure signal invasively. After the dicrotic notch positions have been determined for invasively measured pressure pulses, the dicrotic notch positions can be used for calculating the respiration rate and optionally further physiological parameters. For instance, as also explained above with respective to the non-invasively measured pulse, LVET and/or TPA.sys can be calculated as physiological parameter and also be used for calculating the respiration rate. Also the pulse contour stroke volume (PCSV) as described, for instance, in WO 2019/211210 A1, which is herewith incorporated by reference, could be determined based on the invasive pressure pulse and the dicrotic notch position.

The measured pressure pulses can also be synthesized measured pressure pulses, for instance, pressure pulses which are composed of non-invasively measured tissue pressure pulses or invasively measured tissue pressure pulses. For instance, synthesized pressure pulses can be created from non-invasive pressure pulses with the aim to generate an equivalent of an invasively measured arterial pressure pulse. Therefore, all parameters, that can be determined from an invasively measured arterial pressure pulse can also be determined from a synthesized pressure pulse. In an example, a synthesized pressure pulse can be created by weighting non-invasive pressure pulses and by adding up the weighted the non-invasive pressure pulses. In particular, a synthesized pressure pulse can be generated by weightedly averaging two or more consecutive non-invasive pressure pulses. In an embodiment, a synthesized pressure pulse is generated as described in EP 2 759 258 A1, especially as described in claim 1 of EP 2 759 258 A1, which is herewith incorporated by reference.

Although in above described embodiments as the physiological parameters the respiration rate and optionally the blood pressure have been determined, it is also possible that the apparatus determines further physiological parameters like a fluid responsiveness parameter.

Although in above described embodiments for determining the respiration rate the area under the respective pressure pulse between the start position, at which the respective pressure pulse starts, and the determined dicrotic notch position is used, for determining the respiration rate also other dicrotic-notch-related parameters can be used like the area under the respective pressure pulse between i) a position between the start position and the determined dicrotic notch position, for instance, the position at which the respective pressure pulse reaches its maximum or the position at which the increase, i.e. the first derivative of the pressure pulse, has its maximum, and ii) the dicrotic notch position.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of features of pulses, of certain curves, of the respiration rate, et cetera performed by one or several units or devices can be performed by any other number of units or devices. The calculations and determinations and/or the control of the apparatus for determining the respiration rate of the subject in accordance with the method for determining the respiration rate of the subject can be implemented as program code means of a computer and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims like TPWP_R. t.start, t.notch or t.shift should not be construed as limiting the scope.

The invention relates to an apparatus for determining a respiration rate of a subject. A pressure signal providing unit provides a measured pressure signal of a subject over a time, wherein the measured pressure signal is indicative of blood pulsations and comprises a plurality of pressure pulses, and a processor determines, for a respective pressure pulse of the plurality of pressure pulses, a respective dicrotic notch position and determines the respiration rate based on the dicrotic notch positions determined for the plurality of pressure pulses. This allows for a very accurate determination of the respiration rate.

**Claims**

1. An apparatus for determining a respiration rate of a subject, the apparatus comprising:

   - a pressure signal providing unit configured to provide a measured pressure signal of a subject over a time, wherein the measured pressure signal is indicative of blood pulsations and comprises a plurality of pressure pulses (9),
   - a processor configured to determine, for a respective pressure pulse of the plurality of pressure pulses (9), a respective dicrotic notch position and to determine the respiration rate based on the dicrotic notch positions determined for the plurality of pressure pulses (9).

2. The apparatus as defined by claim 1, wherein the processor is configured to determine, for the respective pressure pulse, a respiration rate determination value (TPWP_R) based on the determined dicrotic notch position such that for several pressure pulses, which are present at different times, several respiration rate determination values (TPWP_R) are determined, wherein the several respiration rate determination values (TPWP _R) determined for the several pressure pulses and hence for several times form a respiration rate determination curve, and to determine the respiration rate based on the respiration rate determination curve.

3. The apparatus as defined by claim 2, wherein the processor is configured to, in order to determine for a respective pressure pulse a respective respiration rate determination value (TPWP_R), determine at least one of:

   - the area under the respective pressure pulse between i) a first position being a start position (t.start), at which the respective pressure pulse starts, or being a position between the start position (t.start) and the determined dicrotic notch position and ii) the determined dicrotic notch position (t.notch), and
   - the difference between the first position and the dicrotic notch position (t.notch).

4. The apparatus as defined by claim 3, wherein the processor is configured such that the respective respiration rate determination value (TPWP _R) is determined by applying a predefined function to at least one of a) the area under the respective pressure pulse between the first position and the determined dicrotic notch position (t.notch), and b) the difference between the first position (t.start) and the dicrotic notch position (t.notch).

5. The apparatus as defined by claim 3, wherein the processor is configured such that the respective respiration rate determination value (TPWP _R) is identical to or linearly related to the area under the respective pressure pulse between the first position, at which the respective pressure pulse starts, and the determined dicrotic notch position (t.notch).

6. The apparatus as defined by any of claims 2 to 5, wherein the processor is configured to determine the respiration rate based on a frequency of the respiration rate determination curve.

7. The apparatus as defined by any of claims 2 to 6, wherein the processor is configured to apply a frequency range pre-selecting filter to the respiration rate determination curve.

8. The apparatus as defined by any of the preceding claims, wherein the processor is configured to determine the respective dicrotic notch position for the respective pressure pulse by determining at least one of

   a) a notch determination intersection position being a position at which a shifted tangent (52) intersects with the pressure pulse, wherein the shifted tangent (52) is determined by determining a tangent (51) to the pressure pulse at a position of the pressure pulse, at which the first derivative of the pressure pulse has a minimum, and by shifting the tangent (51) in the direction of increasing time by a shifting distance (t.shift),
   b) a notch determination maximum position being a position at which a derivative function has a maximum, wherein the derivative function is determined by determining the first derivative of the pressure pulse, by determining the second derivative of the pressure pulse and by combining the determined first and second derivatives, and
   c) a notch determination function position that is determined by determining a characteristic of the pressure pulse and by applying a position determination function to the characteristic, wherein the position determination function provides a relation between the characteristic and the notch determination function position.

9. The processor as defined by claim 8, wherein the processor is configured to provide the shifting distance (t.shift)

such that it is dependent on a characteristic of the pressure pulse (50).

10. The processor as defined by any of claims 8 and 9, wherein the processor is configured to, if several intersection positions of the shifted tangent with the pressure pulse exist, select the temporally last intersection position as the notch determination intersection position.

11. The processor as defined by any of claims 8 to 10, wherein the processor is configured to determine the notch determination maximum position by determining whether the highest maximum of the derivative function is larger than the second highest maximum of the derivative function multiplied by a predefined factor, wherein, if this is the case, the notch determination maximum position is the position of the highest maximum and, if this is not the case, the notch determination maximum position is the position of the first maximum of the derivative function.

12. The processor as defined by any of claims 8 to 11, wherein the processor is configured to determine the derivative function by dividing a) a second derivative function that depends on the second derivative by b) a first derivative function that depends on the first derivative.

13. The processor as defined by any claims 8 to 12, wherein the processor is configured to provide the position determination function such that it provides a relation between the notch determination function position and at least one of the following characteristics of the pressure pulse: the maximum value (Pulse.max) of the pressure pulse (50), the maximum position (t.max) along the pressure pulse (50), a first position (t.dia.pre) along the pressure pulse (50) before the maximum position (t.max) and at which the pressure pulse (50) has a value being a predefined first fraction (p1) of the maximum value (Pulse.max), a second position (t.dia.post) along the pressure pulse (50) behind the maximum position (Pulse.max) and at which the pressure pulse (50) has a value being a predefined second fraction (p2) of the maximum value (Pulse.max), the maximum value (d(Pulse)/dt.max) of the first derivative of the pressure pulse (50), the difference (PP) between the pressure pulse's maximum systolic pressure and the pressure pulse's pressure at the end-diastolic point.

14. A method for determining a respiration rate of a subject, the method comprising:

   - providing a measured pressure signal of a subject over a time by a pressure signal providing unit, wherein the measured pressure signal is indicative of blood pulsations and comprises a plurality of pressure pulses (9),
   - determining, for a respective pressure pulse of the plurality of pressure pulses (9), a respective dicrotic notch position by a processor,
   - determining the respiration rate based on the dicrotic notch positions determined for the plurality of pressure pulses (9).

15. A computer program for determining a respiration rate of a subject, the computer program comprising program code means for causing the apparatus for determining a respiration rate as defined by claim 1 to carry out the steps of the method as defined by claim 14.

FIG. 1

# FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

Europäisches
Patentamt

European
Patent Office

Office européen
des brevets

# EUROPEAN SEARCH REPORT

Application Number

EP 23 15 7442

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/079657 A1 (OCHS JAMES [US] ET AL) 28 March 2013 (2013-03-28) | 1-9, 13-15 | INV. A61B5/08 A61B5/021 A61B5/00 |
| A | * paragraphs [0068], [0072] - [0073], [0080], [0083] - [0084], [0113], [0116], [0127], [0138] - [0142], [0144] * <br> * figures 1-2, 4-7 * | 10-12 | |
| A | DE 10 2017 110770 B3 (UP MED GMBH [DE]) 23 August 2018 (2018-08-23) * the whole document * | 1-15 | |

TECHNICAL FIELDS
SEARCHED (IPC)

A61B

The present search report has been drawn up for all claims

| Place of search | Date of completion of the search | Examiner |
|---|---|---|
| The Hague | 20 July 2023 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 7442

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2023

| Patent document cited in search report | Publication date | Patent family member(s) | Publication date |
|---|---|---|---|
| US 2013079657 A1 | 28-03-2013 | NONE | |
| DE 102017110770 B3 | 23-08-2018 | CN 110913756 A | 24-03-2020 |
| | | DE 102017110770 B3 | 23-08-2018 |
| | | EP 3624683 A1 | 25-03-2020 |
| | | JP 7191093 B2 | 16-12-2022 |
| | | JP 2020520292 A | 09-07-2020 |
| | | WO 2018210931 A1 | 22-11-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011098763 A
- WO 2018210931 A1
- WO 2014121945 A1
- WO 2019211210 A1
- EP 2759258 A1

**Non-patent literature cited in the description**

- **B. SAUGEL et al.** How to measure blood pressure using an arterial catheter: a systematic 5-step approach. *Critical Care,* 2020, vol. 24, 172